# EUROPEAN PATENT APPLICATION

(11) **EP 4 385 461 A1**
(43) Date of publication of application: **19.06.2024**
(21) Application number: 23199189.4
(22) Date of filing: 22.09.2023
(51) Int. Cl.: A61F 2/24

(54) **PROSTHETIC HEART VALVE FRAME STRUCTURE**

(30) Priority: 05.10.2022 US 202263413444 P; 18.09.2023 US 202318468983
(71) Applicant: Medtronic Vascular Inc., Santa Rosa, CA 95403 (US)
(72) Inventor: Kibria, Alkindi, Santa Ana, 92705 (US); Cheshko, Tasha, Santa Ana, 92705 (US); Dorman, Kyle J., Santa Ana, 92705 (US); Goshgarian, Justin G., Santa Rosa, 95403 (US); Dinh, Thuy Linh L., Santa Ana, 92705 (US); Neuberger, Yas B., Santa Rosa, 95403 (US)
(74) Representative: Zimmermann & Partner Patentanwälte mbB

(57) **Abstract**

The techniques of this disclosure generally relate to a valve prosthesis having a prosthetic valve, a stent structure, and anti-migration spikes. The stent structure is coupled to the prosthetic valve and includes an inflow portion. The anti-migration spikes are attached to the inflow portion, the anti-migration spikes protruding radially outward and in an outflow direction from the inflow portion. The anti-migration spikes penetrate into tissue of the vessel, e.g., the aortic annulus of a patient's left ventricle, to prevent migration, i.e., undesirable movement, of the valve prosthesis within the vessel after deployment.

## Description

### CROSS-REFERENCE TO RELATED APPLICATIONS

This application claims the benefit of U.S. Provisional Application No. 63/413,444, filed October 5, 2022, the contents of which are incorporated by reference herein in their entirety.

### FIELD

The present technology is generally related to transcatheter heart valves.

### BACKGROUND

A human heart includes four heart valves that determine the pathway of blood flow through the heart: the mitral valve, the tricuspid valve, the aortic valve, and the pulmonary valve. The mitral and tricuspid valves are atrioventricular valves, which are between the atria and the ventricles, while the aortic and pulmonary valves are semilunar valves, which are in the arteries leaving the heart. Ideally, native leaflets of a heart valve move apart from each other when the valve is in an open position, and meet or "coapt" when the valve is in a closed position. Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

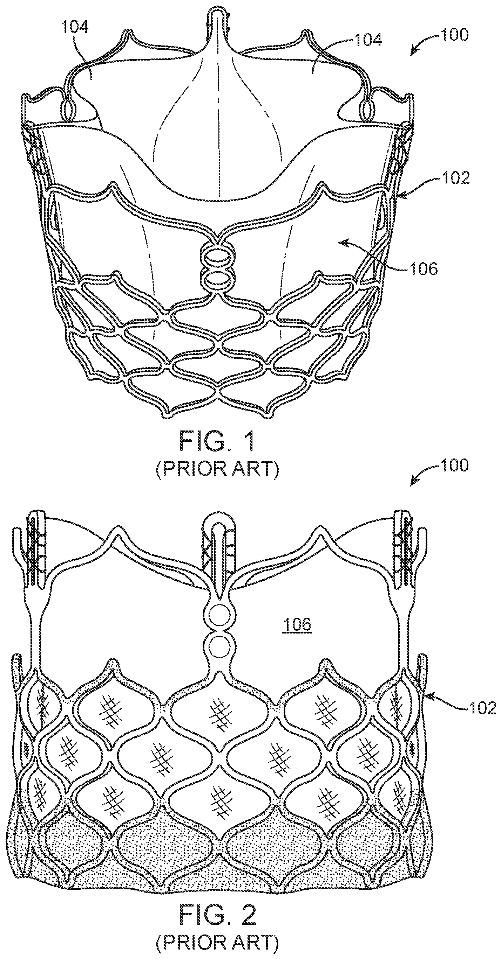

Recently, flexible prosthetic valves supported by stent structures that can be delivered percutaneously using a catheter-based delivery system have been developed for heart and venous valve replacement. FIG. 1 is a perspective view of a valve prosthesis 100 in accordance with the prior art. FIG. 2 is a side view of valve prosthesis 100 of FIG. 1 in accordance with the prior art. Referring to FIGS. 1 and 2, valve prosthesis 100 may include a balloon-expandable stent structure 102 with valve leaflets 104 attached to the interior of stent structure 102. Valve leaflets 104 may be parts of a 3D single piece prosthetic valve 106.

Valve prosthesis 100 can be reduced in diameter, by crimping onto a balloon catheter, and advanced through the venous or arterial vasculature. Once valve prosthesis 100 is positioned at the treatment site, for instance within an incompetent native valve, stent structure 102 may be expanded to hold valve prosthesis 100 firmly in place.

When designing a valve prosthesis such as valve prosthesis 100, valve-frame integration and frame mechanical performance often have competing needs or requirements. For example, when attaching the valve to the frame during valve-frame integration, the valve itself needs to be reinforced to the frame at certain locations without hindering mechanical performance of the frame. Embodiments hereof relate to an improved balloon-expandable transcatheter valve prosthesis configured to minimize tradeoffs between the above-described competing needs.

### SUMMARY

The techniques of this disclosure generally relate to a valve prosthesis having a prosthetic valve, a stent structure, and anti-migration spikes. The stent structure is coupled to the prosthetic valve and includes an inflow portion. The anti-migration spikes are attached to the inflow portion, the anti-migration spikes protruding radially outward and in an outflow direction from the inflow portion.

In one aspect, the present disclosure provides a valve prosthesis having a prosthetic valve, and a stent structure coupled to the prosthetic valve. The valve prosthesis has an hour-glass shape.

In another aspect, the present disclosure provides a method including expanding a balloon to deploy a valve prosthesis comprising a prosthetic valve. The valve prosthesis has an hour-glass shape, the valve prosthesis assuming a shape of the balloon.

In another aspect, the present disclosure provides a valve prosthesis having a prosthetic valve, and a stent structure coupled to the prosthetic valve. The stent structure includes an inflow portion having outflow crowns, an outflow portion including commissure posts having axial frame members and trident posts, and support struts extending between outflow crowns directly adjacent to the commissure posts and to intersections of the axial frame members and the trident posts.

In another aspect, the present disclosure provides a valve prosthesis having a prosthetic valve, a stent structure coupled to the prosthetic valve, and buffer nets. The stent structure includes an outflow portion having commissure posts and non-commissure posts, each non-commissure post being a single circle. The buffer nets are attached to inside surfaces of the non-commissure posts.

In another aspect, the present disclosure provides a valve prosthesis having a prosthetic valve and a stent structure coupled to the prosthetic valve. The stent structure includes an inflow portion comprising outflow crowns. The stent structure further includes an outflow portion having commissure posts and non-commissure posts. All the outflow crowns of the inflow portion are connected to the commissure posts and the non-commissure posts.

In another aspect, the present disclosure provides a valve prosthesis having a prosthetic valve, a stent structure, and valve reinforcement sutures attaching the prosthetic valve to the stent structure. The prosthetic valve includes commissures and a cylindrical inflow end. The valve reinforcement sutures extend longitudinally from the commissures to the cylindrical inflow end.

In another aspect, the present disclosure provides a valve prosthesis having a prosthetic valve and a stent structure coupled to the prosthetic valve. The stent structure has trident posts having an outward slant with respect to a longitudinal axis of the valve prosthesis.

In another aspect, the present disclosure provides a valve prosthesis having a prosthetic valve and a reinforcement member collagen bonded to the prosthetic valve.

In an example, the present disclosure relates to a valve prosthesis including a prosthetic valve, a stent structure coupled to the prosthetic valve, the stent structure comprising an inflow portion; and anti-migration spikes attached to the inflow portion, the anti-migration spikes protruding radially outward and in an outflow direction from the inflow portion.

In another example hereof, in the valve prosthesis according to any of the preceding or following examples, the anti-migration spikes are self-expanding and the stent structure is balloon expandable.

In another example hereof, in the valve prosthesis according to any of the preceding or following examples, the inflow portion comprises central nodes, the anti-migration spikes being attached to the central nodes.

In another example hereof, in the valve prosthesis according to any of the preceding or following examples, the inflow portion comprises first, second, third, and fourth rows of inflow portion cells, and the anti-migration spikes are attached to the inflow portion at the third row of inflow portion cells.

In another example hereof, the valve prosthesis according to any of the preceding or following examples further includes an inflow end outer skirt covering the first, second, and third rows of inflow portion cells.

In another example hereof, in the valve prosthesis according to any of the preceding or following examples, the inflow end outer skirt includes a self-expanding material.

In another example, the present disclosure relates to a valve prosthesis including a prosthetic valve and a stent structure coupled to the prosthetic valve, the valve prosthesis having an hour-glass shape.

In another example hereof, in the valve prosthesis according to any of the preceding or following examples, the valve prosthesis tapers inward from an inflow end of the valve prosthesis to a middle region of the valve prosthesis and tapers outward from the middle region to an outflow end of the valve prosthesis.

In another example hereof, in the valve prosthesis according to any of the preceding or following examples, the valve prosthesis has a first diameter at an inflow end of the valve prosthesis, a second diameter at a middle region of the valve prosthesis, and a third diameter at an outflow end of the valve prosthesis, the second diameter being less than the first diameter and less than the third diameter.

In another example hereof, the present disclosure relates to a method comprising: expanding a balloon to deploy a valve prosthesis comprising a prosthetic valve, wherein upon deployment, the valve prosthesis has an hour-glass shape, the valve prosthesis assuming a shape of the balloon.

In another example hereof, in the method according to any of the preceding or following examples, the balloon has an hourglass shaped outer surface having a diameter at a middle region of the balloon less than a diameter at a distal end of the balloon and less than a diameter at a proximal end of the balloon when inflated.

In another example hereof, in the method according to any of the preceding or following examples, the balloon is hourglass shaped when inflated.

In another example hereof, the present disclosure is related to a valve prosthesis including a prosthetic valve and a stent structure coupled to the prosthetic valve. The stent structure includes: an inflow portion comprising outflow crowns; an outflow portion comprising commissure posts comprising axial frame members and trident posts; and support struts extending between outflow crowns directly adjacent to the commissure posts and to intersections of the axial frame members and the trident posts.

In another example hereof, in the valve prosthesis according to any of the preceding or following examples, the outflow portion consists of the axial frame members and the trident posts.

In another example hereof, the present disclosure is related to a valve prosthesis including a prosthetic valve, a stent structure coupled to the prosthetic valve, the stent structure including an outflow portion including commissure posts and non-commissure posts, and buffer nets attached to inside surfaces of the non-commissure posts. Each non-commissure post includes a single circle.

In another example hereof, in the valve prosthesis according to any of the preceding or following examples, the buffer nets are configured to act as a net to reduce any damaging contact between the prosthetic valve and the non-commissure posts.

In another example hereof, the present disclosure is related to a valve prosthesis including a prosthetic valve and a stent structure coupled to the prosthetic valve. The stent structure includes an inflow portion comprising outflow crowns and an outflow portion. The outflow portion includes commissure posts and non-commissure posts, wherein all the outflow crowns of the inflow portion are connected to the commissure posts and the non-commissure posts.

In another example hereof, in the valve prosthesis according to any of the preceding or following examples, the inflow portion comprises first, second, third, and fourth rows of inflow portion cells, the fourth row of inflow portion cells only being formed directly below the commissure posts and the non-commissure posts.

In another example hereof, the present disclosure is related to a valve prosthesis including a prosthetic valve, a stent structure, and valve reinforcement sutures. The prosthetic valve includes commissures and a cylindrical inflow end. The valve reinforcement sutures attach the prosthetic valve to the stent structure and extend longitudinally from the commissures to the cylindrical inflow end.

In another example hereof, in the valve prosthesis according to any of the preceding or following examples, the valve reinforcement sutures redistribute stress from the commissures over the prosthetic valve.

In another example hereof, in the valve prosthesis according to any of the preceding or following examples, the prosthetic valve has an hour-glass shape.

In another example hereof, in the valve prosthesis according to any of the preceding or following examples, the stent structure includes a flared outflow crown ring.

In another example hereof, in the valve prosthesis according to any of the preceding or following examples, the flared outflow crown ring has a maximum diameter at an outflow end of the stent structure.

In another example hereof, the present disclosure is related to a valve prosthesis including a prosthetic valve and a stent structure coupled to the prosthetic valve. The stent structure includes trident posts having an outward slant with respect to a longitudinal axis of the valve prosthesis.

In another example hereof, in the valve prosthesis according to any of the preceding or following examples, the prosthetic valve has commissures attached to the trident posts.

In another example hereof, the present disclosure is related to a valve prosthesis including a prosthetic valve and a reinforcement member collagen bonded to the prosthetic valve.

In another example hereof, in the valve prosthesis according to any of the preceding or following examples, the prosthetic valve includes valve leaflets and a valve inflow cylinder proximal to the valve leaflets, wherein the reinforcement member is bonded to the valve inflow cylinder.

In another example hereof, in the valve prosthesis according to any of the preceding or following examples, the prosthetic valve includes commissures, wherein the reinforcement member is bonded to the commissures.

Further disclosed herein is a valve prosthesis that has a prosthetic valve, a stent structure, and anti-migration spikes, wherein the stent structure is coupled to the prosthetic valve and includes an inflow portion, wherein the anti-migration spikes are attached to the inflow portion, the anti-migration spikes protruding radially outward and in an outflow direction from the inflow portion, wherein the anti-migration spikes penetrate into tissue of the vessel, e.g., the aortic annulus of a patient's left ventricle, to prevent migration, i.e., undesirable movement, of the valve prosthesis within the vessel after deployment.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of a valve prosthesis in accordance with the prior art.
FIG. 2 is a side view of the valve prosthesis of FIG. 1 in accordance with the prior art.
FIG. 3 is a perspective view of a valve prosthesis in an expanded configuration in accordance with one embodiment.
FIG. 4 is an outflow end view of a prosthetic valve of the valve prosthesis of FIG. 3 in accordance with one embodiment.
FIG. 5 is a perspective view of the prosthetic valve of the valve prosthesis of FIG. 3 in accordance with one embodiment.
FIG. 6 is a side view of the valve prosthesis of FIG. 3 in accordance with one embodiment.
FIG. 7 is a side view of a valve prosthesis in an expanded configuration in accordance with another embodiment.
FIG. 8 is a side view of a valve prosthesis in an expanded configuration in accordance with another embodiment.
FIG. 9 is a side view of a working length of an expanded hour-glass shaped balloon used to deploy the valve prosthesis of FIG. 8 with an hour-glass shape in accordance with one embodiment.
FIG. 10 is a side view of a valve prosthesis in an expanded configuration in accordance with another embodiment.
FIG. 11 is a side view of a valve prosthesis in an expanded configuration in accordance with another embodiment.
FIG. 12 is a side view of a valve prosthesis in an expanded configuration in accordance with another embodiment.
FIG. 13 is a side view of a valve prosthesis in an expanded configuration in accordance with another embodiment.
FIG. 14 is a perspective view of a prosthetic valve of the valve prosthesis of FIG. 13 illustrating valve reinforcement sutures in accordance with one embodiment.
FIG. 15 is a profile view of an outflow portion crown ring as a replacement for an outflow portion crown ring of the valve prosthesis of FIG. 13 in accordance with another embodiment.
FIG. 16 is a side view of a valve prosthesis in an expanded configuration in accordance with another embodiment.
FIG. 17 is a laid flat plan view of a tissue coupon illustrating a prosthetic valve including a reinforcement member in accordance with one embodiment.

### DETAILED DESCRIPTION

Specific embodiments are now described with reference to the figures, wherein like reference numbers indicate identical or functionally similar elements. The terms "distal" and "proximal", when used in the following description to refer to a native vessel, native valve, or a device to be implanted into a native vessel or native valve, such as a heart valve prosthesis, are with reference to the direction of blood flow. Thus, "distal" and "distally", also referred to as the "outflow" or "outflow direction", respectively, refer to positions in a downstream direction with respect to the direction of blood flow. The terms "proximal" and "proximally", also referred to as the "inflow" or "inflow direction", respectively, refer to positions in an upstream direction with respect to the direction of blood flow.

Although the description is in the context of treatment of an aortic heart valve, embodiments may also be used where it is deemed useful in other valved intraluminal sites that are not in the heart. For example, embodiments may be applied to other heart valves or venous valves as well.

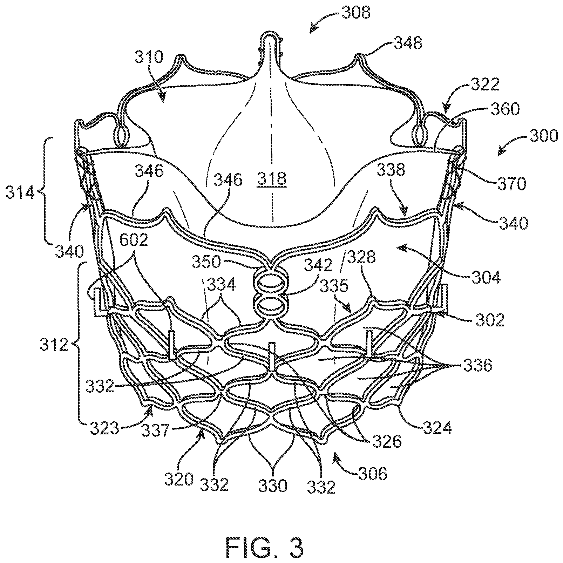

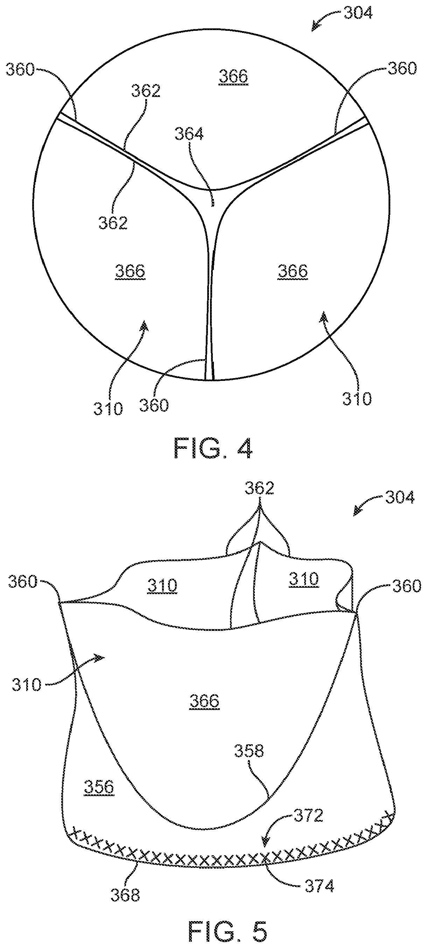

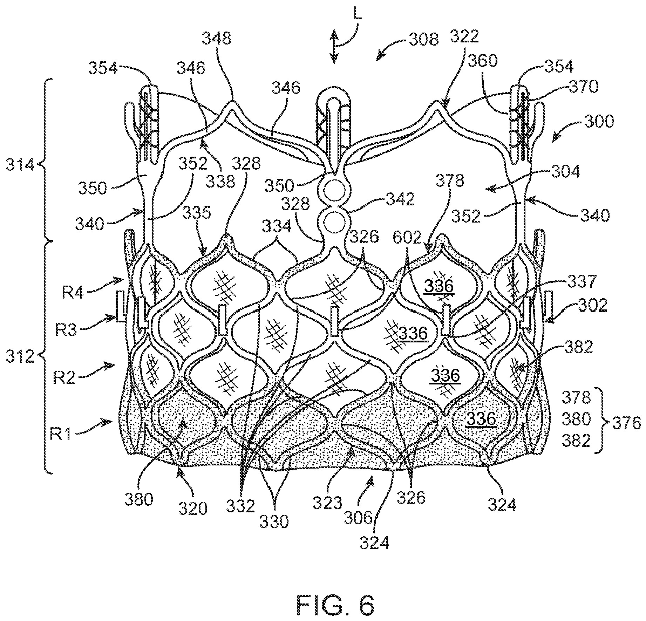

FIG. 3 is a perspective view of a transcatheter valve prosthesis 300 in an expanded configuration in accordance with one embodiment. FIG. 4 is an outflow end view of a prosthetic valve 304 of valve prosthesis 300 of FIG. 3 in accordance with one embodiment. FIG. 5 is a perspective view of prosthetic valve 304 of valve prosthesis 300 of FIG. 3 in accordance with one embodiment. FIG. 6 is a side view of valve prosthesis 300 of FIG. 3 in accordance with one embodiment. FIG. 4 illustrates prosthetic valve 304 in a closed state while FIGS. 3, 5 and 6 illustrates prosthetic valve 304 in an open state.

Referring now to FIGS. 3-6 together, valve prosthesis 300 has a radially-expandable stent structure 302, sometimes called the frame or frame structure, and prosthetic valve 304. Stent structure 302 is generally tubular, and is mechanically or balloon expandable, having a crimped configuration for delivery within a vasculature and an expanded configuration for deployment within a native heart valve. When valve prosthesis 300 is deployed within the valve annulus of a native heart valve, stent structure 302 of valve prosthesis 300 is configured to be radially expanded within native valve leaflets of the patient's defective valve, to thereby retain the native valve leaflets in a permanently open state. In embodiments hereof, valve prosthesis 300 is configured for replacement of an aortic valve such that an inflow end 306, i.e., the proximal end, of valve prosthesis 300 extends into and anchors within the aortic annulus of a patient's left ventricle, while an outflow end 308, i.e., the distal end, of valve prosthesis 300 is positioned within the aortic sinuses.

Stent structure 302 of valve prosthesis 300 may be a unitary frame or scaffold that supports prosthetic valve 304 including one or more valve leaflets 310 within the interior of stent structure 302. Prosthetic valve 304 is capable of blocking flow in one direction to regulate flow there-through via valve leaflets 310 that may form a bicuspid or tricuspid replacement valve. Although prosthetic valve 304 is illustrated and discussed herein as having three valve leaflets 310, i.e., prosthetic valve 304 has a tricuspid leaflet configuration, a bicuspid leaflet configuration may be used in other embodiments. More particularly, as valve prosthesis 300 is configured for placement within a native aortic valve which typically has three leaflets, prosthetic valve 304 may include three valve leaflets 310. However, valve prosthesis 300 is not required to have the same number of leaflets as the native valve. If valve prosthesis 300 is alternatively configured for placement within a native valve having two leaflets such as the mitral valve, prosthetic valve 304 may include two or three valve leaflets 310.

Stent structure 302 is balloon-expandable. As such, stent structure 302 is made from a plastically deformable material such that when expanded by a dilatation balloon, stent structure 302 maintains its radially expanded configuration. Stent structure 302 may be formed from stainless steel such as 316L or other suitable metal, such as platinum iridium, cobalt chromium alloys such as MP35N or L605, or various types of polymers or other similar materials, including the materials coated with various surface deposits to improve clinical functionality. Stent structure 302 is configured to be rigid such that it does not deflect or move when subjected to in-vivo forces, or such that deflection or movement is minimized when subjected to in-vivo forces.

Stent structure 302 includes an inflow portion 312 and an outflow portion 314. Stent structure 302 is a tubular component defining a central lumen or passageway 318, and has an inflow end 320 and an outflow end 322. When expanded, a diameter of inflow end 320 of stent structure 302 is substantially the same as a diameter of outflow end 322 of stent structure 302 in one embodiment.

Inflow portion 312 extends distally from inflow end 320 of stent structure 302. Inflow portion 312 includes inflow crowns 324, central crowns 326, outflow crowns 328, inflow struts 330, central struts 332, and outflow struts 334. Inflow portion 312 further includes an inflow crown ring 323 defined by inflow crowns 324, inflow struts 330, and central crowns 326. Inflow portion 312 further includes an outflow crown ring 335 defined by outflow crowns 328, outflow struts 334, and central crowns 326. Inflow portion 312 generally extends between inflow crown ring 323 and outflow crown ring 335.

In between inflow crown ring 323 and outflow crown ring 335, inflow portion cells 336, sometimes called side openings, are formed in rows, and more particularly, in four rows R1, R2, R3, and R4. Each row R1, R2, R3, R4 includes 12 inflow portion cells 336. Inflow portion cells 336 of the proximal most row R1 are defined by inflow crowns 324, inflow struts 330, central crowns 326, and central struts 332. Inflow portion cells 336 of the next most proximal rows R2, R3 are defined by central crowns 326 and central struts 332. Inflow portion cells 336 of the distal most row R4 are defined by outflow crowns 328, outflow struts 334, central crowns 326, and central struts 332. Generally, inflow portion cells 336 are diamond-shaped openings having the same or identical shaped, e.g., are sometimes called symmetric.

Generally, a crown is defined where two struts connect and a node is defined as a region where two crowns connect. Accordingly, inflow crowns 324 are defined where inflow struts 330 connect. Outflow crowns 328 are defined where outflow struts 334 connect. Central crowns 326 are defined where inflow struts 330 connect, where outflow struts 334 connect, and where central struts 332 connect. Central nodes 337 are defined where central crowns 326 connect.

Outflow portion 314 is formed proximate to outflow end 322 of stent structure 302 and between outflow end 322 and inflow portion 312. Outflow portion 314 includes an outflow portion crown ring 338, commissure posts 340, and non-commissure posts 342. Outflow portion 314 can be configured in a shape that forms a central lumen or passageway.

Outflow portion crown ring 338 includes outflow portion struts 346, superior crowns 348, and inferior crowns 350. Each outflow portion struts 346 is connected at one end to an adjacent outflow crown strut 346 at a superior crown 348 and on the opposite end to an adjacent outflow crown strut 346 at an inferior crown 350. Inferior crowns 350 are connected to either a commissure post 340 or a non-commissure post 342. More particularly, every other inferior crown 350 is connected to a commissure post 340 and every other inferior crown 350 is connected to a non-commissure post 342 in an alternating repeating arrangement.

Non-commissure posts 342 extend longitudinally between and connect inferior crowns 350 of outflow portion crown ring 338 and outflow crowns 328 of inflow portion 312. In accordance with this embodiment, non-commissure posts 342 are shaped as a figure eight and can be used as markers for depth of implant as well as clocking of valve prosthesis 300. As used herein, longitudinally is in a direction parallel with the longitudinal axis L of valve prosthesis 300, radially is perpendicular and in a radial direction from the longitudinal axis L, and circumferentially is in a plane perpendicular to the longitudinal axis L and in a direction along the circumference of valve prosthesis 300.

Commissure posts 340 extend longitudinally and include axial frame members 352 and trident posts 354. Axial frame members 352 extend longitudinally between and connect inferior crowns 350 of outflow portion crown ring 338 and outflow crowns 328 of inflow portion 312. Trident posts 354 extend in a cantilever fashion and in the outflow or distal direction from inferior crowns 350 of outflow portion crown ring 338. Axial frame members 352 and trident posts 354 are parallel with one another and are segments of commissure posts 340, which are linear members.

Prosthetic valve 304 is a one piece 3D molded structure in accordance with this embodiment. Illustratively, a single cylindrical piece is molded to form prosthetic valve 304. Accordingly, although various structures of prosthetic valve 304 are discussed below, prosthetic value 304 is integral, i.e., formed from a single piece and not a plurality of separate pieces connected together.

Prosthetic valve 304 may be made of pericardial material; however, may instead be made of another material. Natural tissue for prosthetic valve 304 may be obtained from, for example, heart valves, aortic roots, aortic walls, aortic leaflets, pericardial tissue, such as pericardial patches, bypass grafts, blood vessels, intestinal submucosal tissue, umbilical tissue and the like from humans or animals. Synthetic materials suitable for use as prosthetic valve 304 include DACRON polyester commercially, other cloth materials, nylon blends, polymeric materials, and vacuum deposition nitinol fabricated materials. One polymeric material from which prosthetic valve 304 can be made is an ultra-high molecular weight polyethylene material. With certain materials, it may be desirable to coat one or both sides of prosthetic valve 304 with a material that will prevent or minimize overgrowth. It is further desirable that the material is durable and not subject to stretching, deforming, or fatigue.

Prosthetic valve 304 includes valve leaflets 310 and a valve inflow cylinder 356 proximal of valve leaflets 310. For example, valve inflow cylinder 356 is the remaining unmolded portion of the cylindrical material used to form prosthetic valve 304 and valve leaflets 310 are the molded portion.

Valve leaflets 310 are defined by cusps 358, commissures 360, and free edges 362. Adjoining pairs of valve leaflets 310 are attached to one another at their lateral ends to form commissures 360, with free edges 362 of valve leaflets 310 forming coaptation edges that meet in an area of coaptation 364. The region within cusps 358, commissures 360, and free edges 362 are referred to as a belly 366 of valve leaflets 310.

Valve inflow cylinder 356 has a cylindrical inflow end 368, sometimes called a nadir 368. Valve inflow cylinder 356 extends in the outflow direction from inflow end 368 as a cylinder to cusps 358, which form the outflow end of valve inflow cylinder 356.

Prosthetic valve 304 is disposed within and secured to at least trident posts 354 of commissure posts 340 of stent structure 302. In addition, prosthetic valve 304 may also be disposed within and secured to inflow portion 312 of stent structure 302. More particularly, commissures 360 are attached to trident posts 354, e.g., with commissure stitching 370. Further, a margin of attachment (MOA) 372 of valve inflow cylinder 356, e.g., a region directly adjacent cylindrical inflow end 368, is attached to inflow portion 312, e.g., with MOA stitching 374.

Valve prosthesis 300 further includes a skirt 376, which encloses or lines a portion of stent structure 302. Skirt 376 is not illustrated in FIG. 3 for clarity of visualization of stent structure 302. Margin of attachment 372 of valve inflow cylinder 356 is sutured or otherwise securely and sealingly attached to the interior surface of skirt 376 and to inflow portion 312, e.g., with MOA stitching 374.

Skirt 376 may enclose or line stent structure 302. Skirt 376 may be a natural or biological material such as pericardium or another membranous tissue such as intestinal submucosa. Alternatively, skirt 376 may be a low-porosity woven fabric, such as polyester, Dacron fabric, or PTFE. In one embodiment, skirt 376 may be a knit or woven polyester, such as a polyester or PTFE knit, which can be utilized when it is desired to provide a medium for tissue ingrowth and the ability for the fabric to stretch to conform to a curved surface. Polyester velour fabrics may alternatively be used, such as when it is desired to provide a medium for tissue ingrowth on one side and a smooth surface on the other side.

In accordance with this embodiment, skirt 376 includes an outflow end outer skirt 378, and inflow end outer skirt 380, and an inner skirt 382. Outflow end outer skirt 378 is located on the outer surface of outflow crowns 328 and outflow struts 334 of inflow portion 312. Inflow end outer skirt 380 is located on the outer surface of inflow crowns 324, inflow struts 330, central crowns 326, and central struts 332. More particularly, inflow end outer skirt 380 covers first row R1 of inflow portion cells 336. Inner skirt 382 is located on the inner surface of inflow portion 312 and extends between inflow crown ring 323 and outflow crown ring 335.

Delivery of valve prosthesis 300 may be accomplished via a percutaneous transfemoral approach or a transapical approach directly through the apex of the heart via a thoracotomy, or may be positioned within the desired area of the heart via different delivery methods known in the art for accessing heart valves. During delivery, valve prosthesis 300 remains compressed until it reaches a target diseased native heart valve, at which time a balloon of a delivery system is inflated in order to radially expand valve prosthesis 300 in situ. Valve prosthesis 300 is configured to be expanded within the native valve leaflets of the patient's defective valve, to thereby retain the native valve leaflets in a permanently open state. The delivery system is then removed and transcatheter valve prosthesis 300 remains deployed within the native target heart valve.

Paying particular attention now to FIGS. 3 and 6, valve prosthesis 300 further includes anti-migration spikes 602. Anti-migration spikes 602 protrude radially outward and in the outflow direction from inflow portion 312 of stent structure 302. Anti-migration spikes 602 penetrate into tissue of the vessel, e.g., the aortic annulus of a patient's left ventricle, to prevent migration, i.e., undesirable movement, of valve prosthesis 300 within the vessel after deployment.

Anti-migration spikes 602 are self-expanding in one embodiment. For example, anti-migration spikes 602 are nitinol or other memory material. Anti-migration spikes 602 are attached to inflow portion 312, e.g., by rivets, welding, adhesive, or other attachment mechanisms. In one embodiment, anti-migration spikes 602 are held in a collapsed delivery configuration against inflow portion 312 during delivery, e.g., by a sheath of the delivery system. Upon reaching the deployment location, the sheath is withdrawn to expose anti-migration spikes 602, which self-expand to protrude radially outward and distally upon being exposed. Valve prosthesis 300 is then balloon expanded as discussed above.

In this embodiment, anti-migration spikes 602 are attached to inflow portion 312 at third row R3 of inflow portion cells 336. More particularly, anti-migration spikes 602 are attached to central nodes 337 of the third row R3 of inflow portion cells 336. Accordingly, anti-migration spikes 602 extend as a row around the entire circumference of valve prosthesis 300 at a particular longitudinal position. However, in other embodiments, anti-migration spikes 602 are attached at other location, e.g., at more than one row around the entire circumference of valve prosthesis 300 or as an array at varying longitudinal and circumferential positions.

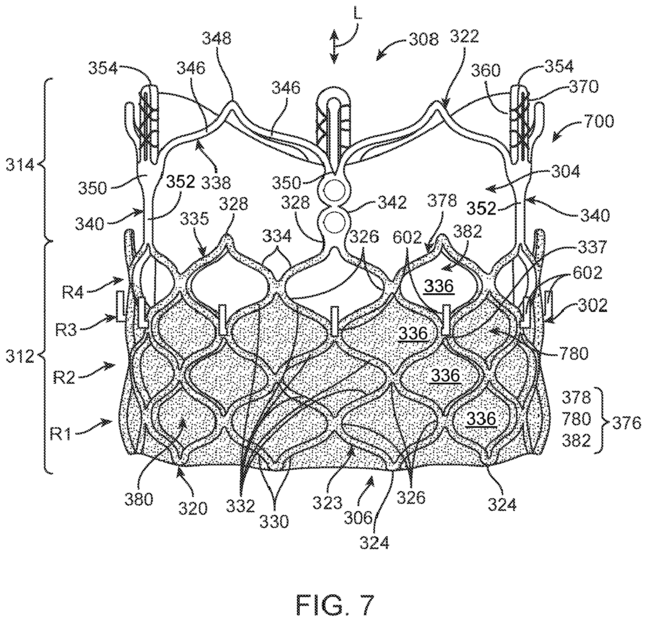

FIG. 7 is a side view of a valve prosthesis 700 in an expanded configuration in accordance with another embodiment. Valve prosthesis 700 of FIG. 7 is similar to valve prosthesis 300 of FIGS. 3-6 and only the significant differences are discussed below.

Referring now to FIG. 7, in accordance with this embodiment, an inflow end outer skirt 780 (instead of inflow end outer skirt 380 of FIG. 6) is located on the outer surface of inflow crowns 324, inflow struts 330, central crowns 326, and central struts 332. More particularly, inflow end outer skirt 780 covers first row R1, second row R2, and third row R3 of inflow portion cells 336. Inflow end outer skirt 780 is sometimes called a paravalvular leak (PVL) wrap. Anti-migration spikes 602 are uncovered by inflow end outer skirt 780.

By increasing the height of the inflow end outer skirt 780 to cover three rows R1, R2, R3 of inflow portion cells 336, there is additional sealing with the vessel in which valve prosthesis 700 is deployed. This additional sealing prevents leakage around inflow portion 312 as well are mitigates migration.

Anti-migration spikes 602 are uncovered by inflow end outer skirt 780. In another embodiment, valve prosthesis 700 of FIG. 7 does not include anti-migration spikes 602 as inflow end outer skirt 780 provides sufficient anti-migration mitigation.

Inflow end outer skirt 780 is material that enhances the sealing capability of inflow portion 312. In various embodiment, inflow end outer skirt 780 includes natural or synthetic materials such as those described above for prosthetic valve 304. In one embodiment, inflow end outer skirt 780 includes a self-expanding material, e.g., strips, coils, and/or threads of nitinol, that expand outwards thus enhancing the sealing capability of inflow end outer skirt 780.

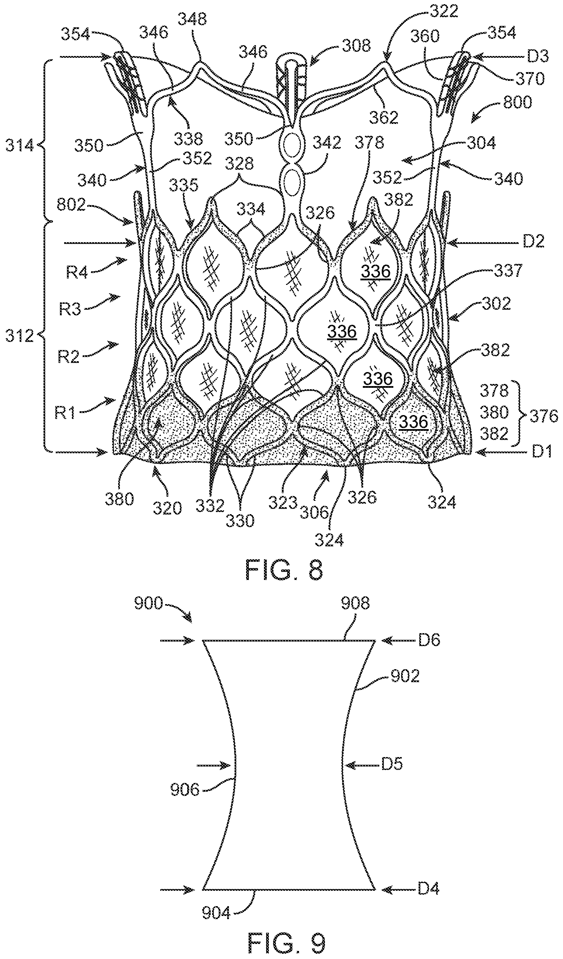

FIG. 8 is a side view of a valve prosthesis 800 in an expanded configuration in accordance with another embodiment. Valve prosthesis 800 of FIG. 8 is similar to valve prosthesis 300 of FIG. 3-6 and only the significant differences are discussed below.

In accordance with this embodiment, valve prosthesis 800 has an hour-glass shape to reduce or eliminate pinwheeling of prosthetic valve 304. More particularly, in the longitudinal direction, valve prosthesis 800 tapers radially inward from inflow end 306 to a middle region 802 of valve prosthesis 800 and tapers radially outward from middle region 802 of valve prosthesis 800 to outflow end 308. Valve prosthesis 800 has a first diameter D1 at inflow end 306, a minimum second diameter D2 at a middle region 802 of valve prosthesis 800, and a third diameter D3 at outflow end 308. Second diameter D2 is less than first diameter D1 and less than third diameter D3. In one embodiment, first diameter D1 is approximately equal to third diameter D3.

Middle region 802 is in the approximate center of valve prosthesis 800 in the longitudinal direction in one embodiment, e.g., at third or fourth row R3, R4 of inflow portion cells 336. However, in other embodiments, middle region 802 is offset towards either inflow end 306 or outflow end 308.

The hour-glass shape of valve prosthesis 800 expands stent structure 302, sometimes called the frame, to open the top section of stent structure 302. For example, the top section is outflow portion crown ring 338 including superior crowns 348 and trident posts 354.

As valve prosthesis 800 has an hour-glass shape, commissures 360 are stretched apart from one another minimizing the amount of extra material of prosthetic valve 304 at free edges 362 of valve leaflets 310. By minimizing the amount of extra material of prosthetic valve 304 at free edges 362 of valve leaflets 310, pinwheeling during opening and closing of prosthetic valve 304 is minimized. This, in turn, reduces stress and the associated damage/failure of prosthetic valve 304.

FIG. 9 is a side view of a working length of an expanded hour-glass shaped balloon 900 used to deploy valve prosthesis 800 of FIG. 8 with an hour-glass shape in accordance with one embodiment. Referring to FIGS. 8 and 9 together, to provide valve prosthesis 800 with an hour-glass shape, valve prosthesis 800 is deployed using hour-glass shaped balloon 900. Hour-glass shaped balloon 900 has an hourglass shaped outer surface 902 having fourth diameter D4 at a distal end 904, a fifth diameter D5 in a middle region 906, and a sixth diameter D6 at a proximal end 908. Fifth diameter D5 is less than fourth diameter D4 and less than sixth diameter D6. The distal end of a delivery system including tapered balloon 900 is usually identified to the end that is farthest from the operator/handle while the proximal end is the end nearest the operator/handle.

As hour-glass shaped balloon 900 is inflated to expand valve prosthesis 800 to the form illustrated in FIG. 8, the hour-glass shape of tapered balloon 900 causes stent structure 302, and thus valve prosthesis 800, to assume the hour-glass shape.

In another embodiment, stent structure 302 has a frame design that allows stent structure 302, and thus valve prosthesis 800, to assume the hour-glass shape, for example, when inflated with a cylindrical balloon. For example, outflow portion crown ring 338 and inflow crown ring 323 are designed to be weaker and/or have longer struts than stent structure 302 at middle region 802. This allows outflow portion crown ring 338 and inflow crown ring 323 to selectively expand relative to middle region 802 thus providing the hour-glass shape.

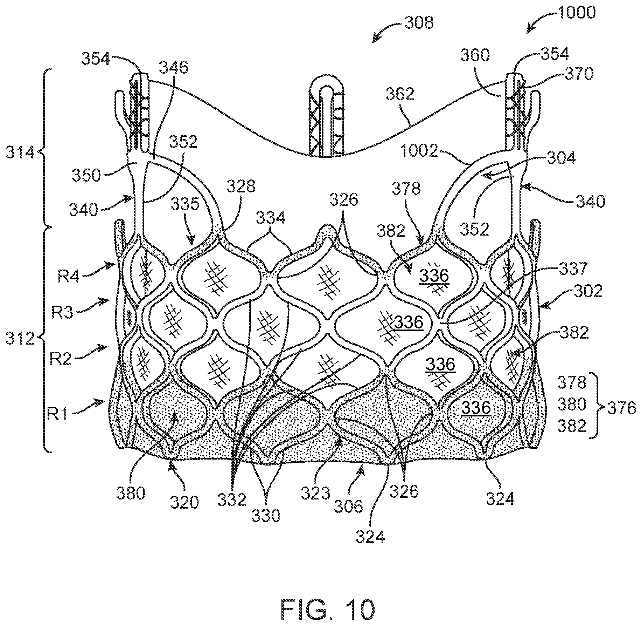

FIG. 10 is a side view of a valve prosthesis 1000 in an expanded configuration in accordance with another embodiment. Valve prosthesis 1000 of FIG. 10 is similar to valve prosthesis 300 of FIG. 3-6 and only the significant differences are discussed below.

Referring now to FIGS. 6 and 10 together, valve prosthesis 1000 is formed without outflow portion crown ring 338 and non-commissure posts 342 of valve prosthesis 300. Further, valve prosthesis 1000 includes supports struts 1002 the provide support to commissure posts 340. More particularly, support struts 1002 extend between outflow crowns 328 that are directly adjacent commissure posts 340 and to commissure posts 340 at the intersection of axial frame members 352 and trident posts 354.

By eliminating outflow portion crown ring 338 and non-commissure posts 342, contact between free edges 362 of valve leaflets 310 and outflow portion crown ring 338/non-commissure posts 342, and the associated damage, is avoided. Further, coronary access is increased.

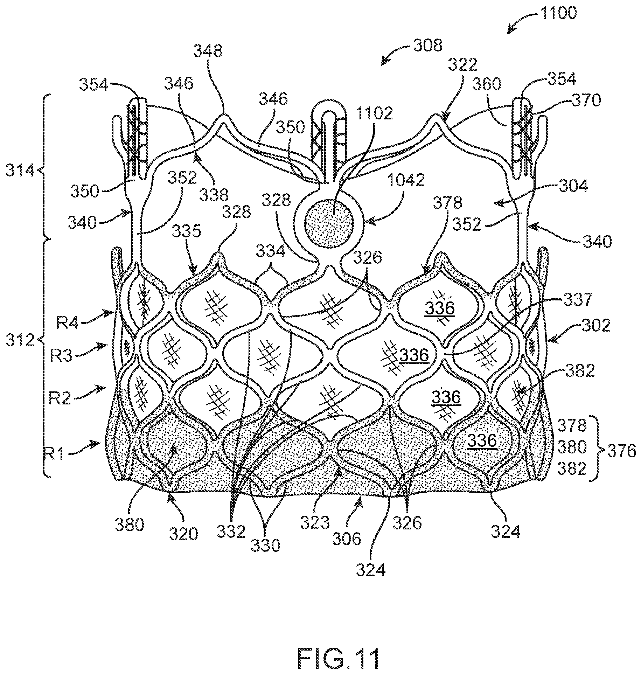

FIG. 11 is a side view of a valve prosthesis 1100 in an expanded configuration in accordance with another embodiment. Valve prosthesis 1100 of FIG. 11 is similar to valve prosthesis 300 of FIG. 3-6 and only the significant differences are discussed below.

Referring now to FIGS. 6 and 11 together, valve prosthesis 1100 is formed with non-commissure posts 1042 instead of non-commissure posts 342 of valve prosthesis 300. More particularly, non-commissure posts 1042 are formed to have a single large circle in contrast to the figure 8 design of non-commissure posts 342 of valve prosthesis 300. Further, a buffer net 1102 is attached to the inside surface of non-commissure posts 1042. In one embodiment, buffer net 1102 is formed of materials similar to those described above for skirt 376 and acts as a net.

By forming non-commissure posts 1042 as a large circle and lining the circle with buffer net 1102, in the event of contact between valve leaflets 310 and non-commissure posts 1042, the blow, e.g.., damaging contact, between the valve leaflets 310 and non-commissure posts 1042 is reduced. This minimizes any damage to valve leaflets 310 from contact with non-commissure posts 1042.

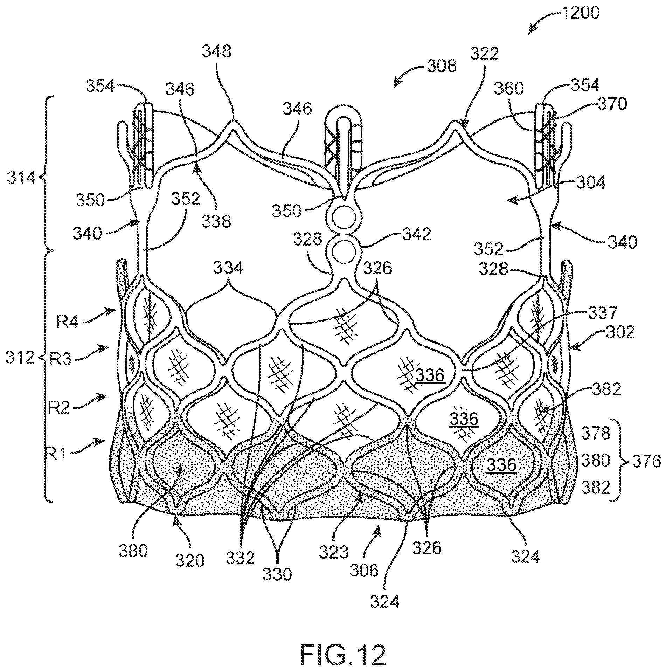

FIG. 12 is a side view of a valve prosthesis 1200 in an expanded configuration in accordance with another embodiment. Valve prosthesis 1200 of FIG. 12 is similar to valve prosthesis 300 of FIG. 3-6 and only the significant differences are discussed below.

Referring now to FIGS. 6 and 12 together, valve prosthesis 1200 is formed without outflow crowns 328 that are radially aligned with superior crowns 348 and the associated outflow struts 334 of valve prosthesis 300. More particularly, each outflow crown 328 that is not connected to a commissure post 340 or a non-commissure post 342, sometimes called a free outflow crown 328, and the two outflow struts 334 that extend to the free outflow crown 328 are removed in valve prosthesis 1200 as compared to valve prosthesis 300. Accordingly, all outflow crowns 328 are connected to either a commissure post 340 or a non-commissure post 342.

In other words, inflow portion cells 336 in fourth row R4 directly below superior crowns 348 are removed. All of the remaining inflow portions cells 336 in fourth row R4 are directly below either a commissure post 340 or a non-commissure post 342, i.e., for a total of six inflow portion cells 336 in fourth row R4 in this embodiment.

By eliminating inflow portion cells 336 in fourth row R4 directly below superior crowns 348, space is opened up in stent structure 302 and coronary access is increased. In addition, expansion symmetry of stent structure 302 is improved in the case where the removed inflow portion cells 336 do not expand as much as the other inflow portion cells 336.

Further, by removing the free outflow crowns 328 directly below superior crowns 348, contact between valve leaflets 310, e.g., bellies 366 thereof, and free outflow crowns 328, and the associated damage, is avoided.

In another embodiment, the frame modifications of FIGS. 11 and 12, and/or any other embodiments, are combined.

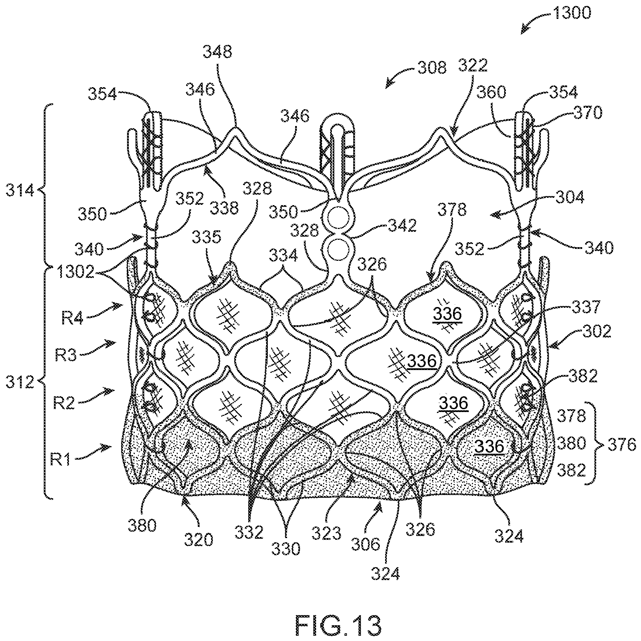

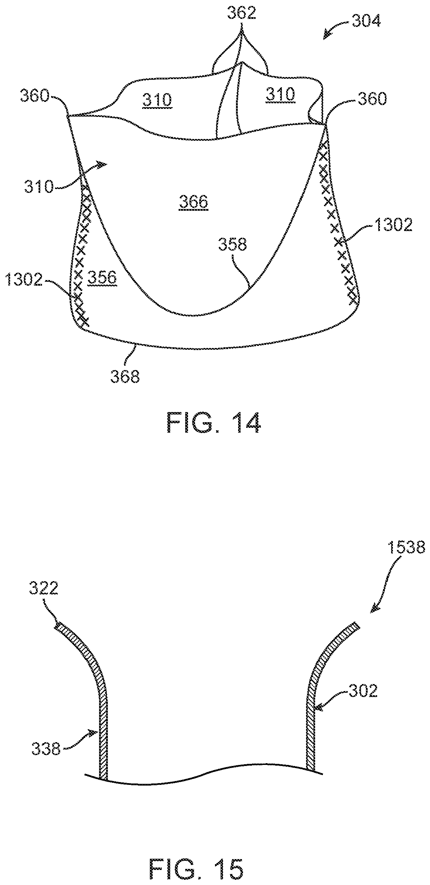

FIG. 13 is a side view of a valve prosthesis 1300 in an expanded configuration in accordance with another embodiment. Valve prosthesis 1300 of FIG. 13 is similar to valve prosthesis 300 of FIG. 3-6 and only the significant differences are discussed below. FIG. 14 is a perspective view of prosthetic valve 304 of valve prosthesis 1300 of FIG. 13 illustrating valve reinforcement sutures 1302 in accordance with one embodiment.

Referring now to FIGS. 13 and 14 together, prosthetic valve 304 is attached to stent structure 302 by valve reinforcement sutures 1302. Valve reinforcement sutures 1302 extend longitudinally along prosthetic valve 304 from commissures 360 to cylindrical inflow end 368 in this embodiment.

Valve reinforcement sutures 1302 are attached to one or more of axial frame members 352 of commissure posts 340, inner skirt 382, outflow crowns 328, central crowns 326, and/or central nodes 337. Accordingly, prosthetic valve 304 is attached to one or more of axial frame members 352 of commissure posts 340, inner skirt 382, outflow crowns 328, central crowns 326, and/or central nodes 337. This attachment of prosthetic valve 304 redistributes stress from commissures 360 over the entirety of the hour-glass shape of prosthetic valve 304. This reduces stress on the attachment of commissures 360 by commissure stitching 370 to trident posts 354.

FIG. 15 is a profile view of an outflow portion crown ring 1538 as a replacement for outflow portion crown ring 338 of valve prosthesis 1300 of FIG. 13 in accordance with another embodiment. As illustrated in FIG. 15, outflow portion crown ring 1538 is flared outward to have a maximum diameter at outflow end 322 of stent structure 302 in accordance with this embodiment. By flaring outflow portion crown ring 1538, contact between valve leaflets 310 and outflow portion crown ring 1538 is minimized or eliminated, as is the associated possible damage of valve leaflets 310.

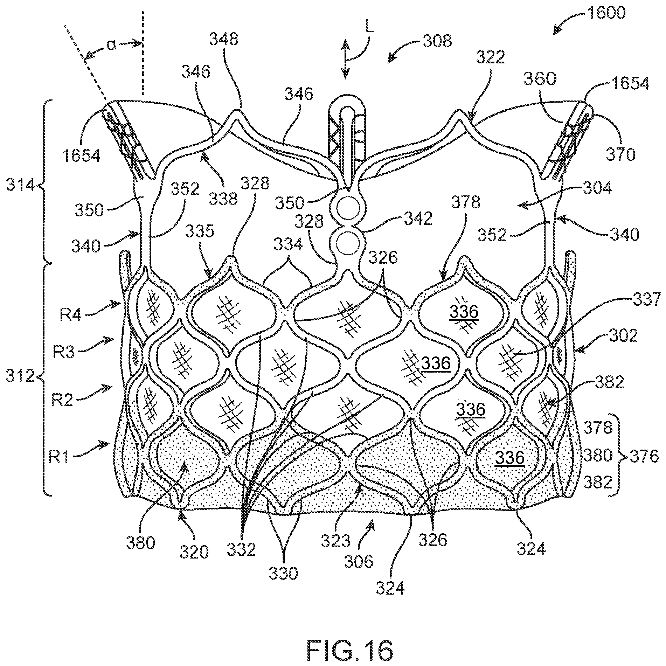

FIG. 16 is a side view of a valve prosthesis 1600 in an expanded configuration in accordance with another embodiment. Valve prosthesis 1600 of FIG. 16 is similar to valve prosthesis 300 of FIG. 3-6 and only the significant differences are discussed below.

In accordance with this embodiment, trident posts 1654 slant outward as compared to longitudinally extending trident posts 354 of valve prosthesis 300. More particularly, trident posts 1654 form a non-zero angle a with respect to longitudinal axis L of valve prosthesis 1600. The outward slant of trident posts 1654 matches the hour-glass shape of prosthetic valve 304 and the profile of commissures 360.

Commissures 360 are attached to slanted trident posts 1654. By matching the profile of commissures 360 with the slant of trident posts 1654, stress on commissures 360 from attachment to trident posts 1654 is reduced as compared to longitudinally extending trident posts 354.

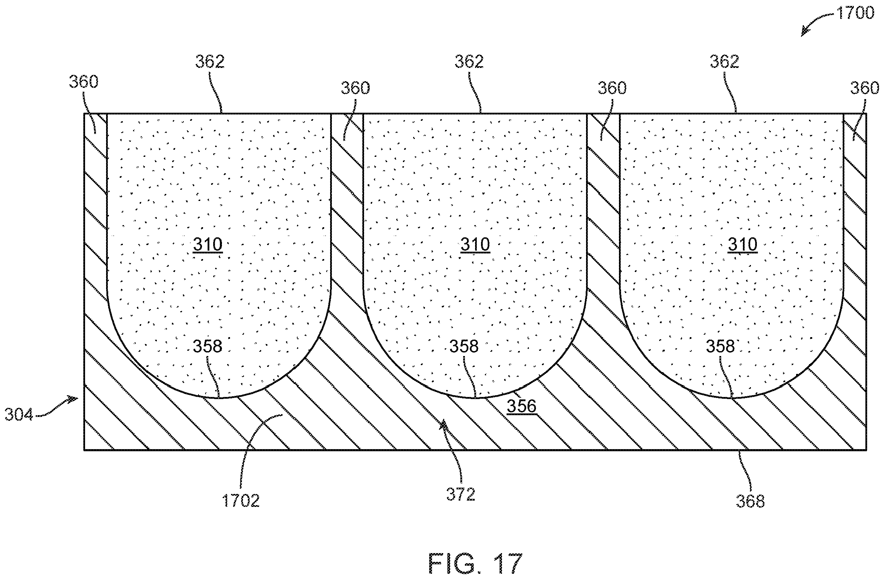

FIG. 17 is a laid flat plan view of a tissue coupon 1700 illustrating prosthetic valve 304 including a reinforcement member 1702 in accordance with one embodiment. Tissue coupon 1700 of FIG. 17 corresponds to prosthetic valve 304 of FIG. 5 cut longitudinally at a commissure 360 with the addition of reinforcement member 1702. It is to be understood that tissue coupon 1700 is set forth in FIG. 17 to facilitate understanding of the structure of prosthetic valve 304 and reinforcement member 1702 but that prosthetic valve 304 is fabricated from a cylindrical piece of material and tissue coupon 1700 would not exist during actual fabrication.

Referring now to FIGS. 5 and 17 together, to provide reinforcement of prosthetic valve 304, reinforcement member 1702 is attached to prosthetic valve 304. In one embodiment, reinforcement member 1702 is pericardial material that is collagen bonded to prosthetic valve 304 by compression.

In the embodiment illustrated, reinforcement member 1702 is bonded proximally of cusps 358 and does not cover valve leaflets 310. Reinforcement member 1702 is bonded to valve inflow cylinder 356 including MOA 372 and around commissures 360. Reinforcement member 1702 creates a rigid structure that aids in keeping the shape of valve inflow cylinder 356 including MOA 372 and commissures 360 thus improving the durability of prosthetic valve 304. Reinforcement member 1702 stiffens commissures 360, particularly the region that gets attached to stent structure 302.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

Further disclosed herein is the subject-matter of the following clauses:
1. A valve prosthesis comprising:
   a prosthetic valve; and
   a stent structure coupled to the prosthetic valve, the stent structure comprising an inflow portion having a plurality of rows of inflow portion cells and an outflow portion comprising a plurality of commissure posts, a plurality of non-commissure posts, and an outflow crown ring coupled to distal ends of the non-commissure posts and intersecting the commissure posts such that trident posts extend distally of a location where the distal crown ring intersects the commissure posts.
2. The valve prosthesis of Clause 1, further comprising anti-migration spikes attached to the inflow portion, the anti-migration spikes protruding radially outward and in an outflow direction from the inflow portion.
3. The valve prosthesis of Clause 2 wherein the anti-migration spikes are self-expanding and the stent structure is balloon expandable.
4. The valve prosthesis of Clause 2 or of any of Clauses 2-3, wherein the inflow portion cells are formed by rows of struts and crowns, wherein crowns of adjacent rows of struts and crowns form nodes, and wherein the anti-migration spikes are attached to the nodes.
5. The valve prosthesis of Clause 4 wherein the inflow portion cells comprise first, second, third, and fourth rows of inflow portion cells, and wherein the anti-migration spikes are attached to the inflow portion at the third row of inflow portion cells.
6. The valve prosthesis of Clause 5 further comprising an inflow end outer skirt covering the first, second, and third rows of inflow portion cells.
7. The valve prosthesis of Clause 6 wherein the inflow end outer skirt includes a self-expanding material.
8. The valve prosthesis of Clause 1 or of any of the preceding Clauses, the valve prosthesis having an hourglass shape.
9. The valve prosthesis of Clause 8 wherein the valve prosthesis has a first diameter at an inflow end of the valve prosthesis, a second diameter at a middle region of the valve prosthesis, and a third diameter at an outflow end of the valve prosthesis, the second diameter being less than the first diameter and less than the third diameter.
10. The valve prosthesis of Clause 1 or of any of the preceding Clauses, wherein the non-commissure posts include a single circle, further comprising buffer nets attached to inside surfaces of the non-commissure posts, wherein the buffer nets are configured to reduce damaging contact between the prosthetic valve and the non-commissure posts.
11. The valve prosthesis of Clause 1 or of any of the preceding Clauses, wherein the prosthetic valve comprises commissures and a cylindrical inflow end, further comprising valve reinforcement sutures attaching the prosthetic valve to the stent structure, the valve reinforcement sutures extending longitudinally from the commissures to the cylindrical inflow end.
12. The valve prosthesis of Clause 11 wherein the prosthetic valve has an hourglass shape.
13. The valve prosthesis of Clause 1 or of any of the preceding Clauses, wherein the trident posts have an outward slant with respect to a longitudinal axis of the valve prosthesis, and wherein the prosthetic valve includes commissures attached to the trident posts.
14. The valve prosthesis of Clause 1 or of any of the preceding Clauses, wherein the prosthetic valve comprises valve leaflets and a valve inflow cylinder proximal to the valve leaflets, and further comprising a reinforcement member collagen bonded to the valve inflow cylinder or to commissures of the valve leaflets.
15. A valve prosthesis comprising:
   a prosthetic valve; and
   a stent structure coupled to the prosthetic valve, the stent structure comprising:
      an inflow portion comprising a plurality of rows of inflow portion cells, the inflow portion comprising outflow crowns defining a distalmost row of cells of the plurality of rows;
      an outflow portion comprising commissure posts comprising axial frame members and trident posts extending distally from the axial frame members; and
      support struts extending between outflow crowns directly adjacent to the commissure posts and to intersections of the axial frame members and the trident posts.
16. The valve prosthesis of Clause 15 wherein the outflow portion consists of the axial frame members and the trident posts.
17. A valve prosthesis comprising:
   a prosthetic valve; and
   a stent structure coupled to the prosthetic valve, the stent structure comprising:
      an inflow portion comprising a plurality of rows of inflow portion cells, the inflow portion comprising outflow crowns defining a distalmost row of cells of the plurality of rows; and
      an outflow portion comprising:
         commissure posts; and
         non-commissure posts disposed circumferentially between adjacent commissure posts,
   wherein each outflow crown of the inflow portion is attached to a corresponding one of the commissure posts or a corresponding one of the non-commissure posts.
18. The valve prosthesis of Clause 17 wherein the inflow portion comprises first, second, third, and fourth rows of inflow portion cells, the fourth row of inflow portion cells only being formed directly below the commissure posts and the non-commissure posts.
19. The valve prosthesis of Clause 17, wherein the inflow portion comprises a plurality of inflow crowns defining an inflow end of the valve prosthesis, wherein the inflow portion comprises double the number of inflow crowns as outflow crowns.
20. The valve prosthesis of Clause 19, wherein the inflow portion includes exactly twelve inflow crowns and exactly six outflow crowns.

## Claims

1. A valve prosthesis comprising:
a prosthetic valve; and
a stent structure coupled to the prosthetic valve, the stent structure comprising an inflow portion having a plurality of rows of inflow portion cells and an outflow portion comprising a plurality of commissure posts, a plurality of non-commissure posts, and an outflow crown ring coupled to distal ends of the non-commissure posts and intersecting the commissure posts such that trident posts extend distally of a location where the distal crown ring intersects the commissure posts.

2. The valve prosthesis of Claim 1, further comprising anti-migration spikes attached to the inflow portion, the anti-migration spikes protruding radially outward and in an outflow direction from the inflow portion.

3. The valve prosthesis of Claim 2 wherein the anti-migration spikes are self-expanding and the stent structure is balloon expandable.

4. The valve prosthesis of any of Claims 2-3 wherein the inflow portion cells are formed by rows of struts and crowns, wherein crowns of adj acent rows of struts and crowns form nodes, and wherein the anti-migration spikes are attached to the nodes.

5. The valve prosthesis of Claim 4 wherein the inflow portion cells comprise first, second, third, and fourth rows of inflow portion cells, and wherein the anti-migration spikes are attached to the inflow portion at the third row of inflow portion cells.

6. The valve prosthesis of Claim 5 further comprising an inflow end outer skirt covering the first, second, and third rows of inflow portion cells, wherein optionally the inflow end outer skirt includes a self-expanding material.

7. The valve prosthesis of any of the preceding Claims, the valve prosthesis having an hourglass shape, wherein optionally the valve prosthesis has a first diameter at an inflow end of the valve prosthesis, a second diameter at a middle region of the valve prosthesis, and a third diameter at an outflow end of the valve prosthesis, the second diameter being less than the first diameter and less than the third diameter.

8. The valve prosthesis of any of the preceding Claims, wherein the non-commissure posts include a single circle, further comprising buffer nets attached to inside surfaces of the non-commissure posts, wherein the buffer nets are configured to reduce damaging contact between the prosthetic valve and the non-commissure posts.

9. The valve prosthesis of any of the preceding Claims, wherein the prosthetic valve comprises commissures and a cylindrical inflow end, further comprising valve reinforcement sutures attaching the prosthetic valve to the stent structure, the valve reinforcement sutures extending longitudinally from the commissures to the cylindrical inflow end, wherein optionally the prosthetic valve has an hourglass shape.

10. The valve prosthesis of any of the preceding Claims, wherein the trident posts have an outward slant with respect to a longitudinal axis of the valve prosthesis, and wherein the prosthetic valve includes commissures attached to the trident posts.

11. The valve prosthesis of any of the preceding Claims, wherein the prosthetic valve comprises valve leaflets and a valve inflow cylinder proximal to the valve leaflets, and further comprising a reinforcement member collagen bonded to the valve inflow cylinder or to commissures of the valve leaflets.

12. A valve prosthesis comprising:
a prosthetic valve; and
a stent structure coupled to the prosthetic valve, the stent structure comprising:
an inflow portion comprising a plurality of rows of inflow portion cells, the inflow portion comprising outflow crowns defining a distalmost row of cells of the plurality of rows;
an outflow portion comprising commissure posts comprising axial frame members and trident posts extending distally from the axial frame members; and
support struts extending between outflow crowns directly adjacent to the commissure posts and to intersections of the axial frame members and the trident posts, wherein optionally the outflow portion consists of the axial frame members and the trident posts.

13. A valve prosthesis comprising:
a prosthetic valve; and
a stent structure coupled to the prosthetic valve, the stent structure comprising:
an inflow portion comprising a plurality of rows of inflow portion cells, the inflow portion comprising outflow crowns defining a distalmost row of cells of the plurality of rows; and
an outflow portion comprising:
commissure posts; and
non-commissure posts disposed circumferentially between adjacent commissure posts,
wherein each outflow crown of the inflow portion is attached to a corresponding one of the commissure posts or a corresponding one of the non-commissure posts.

14. The valve prosthesis of Claim 13 wherein the inflow portion comprises first, second, third, and fourth rows of inflow portion cells, the fourth row of inflow portion cells only being formed directly below the commissure posts and the non-commissure posts.

15. The valve prosthesis of Claim 13, wherein the inflow portion comprises a plurality of inflow crowns defining an inflow end of the valve prosthesis, wherein the inflow portion comprises double the number of inflow crowns as outflow crowns, wherein optionally the inflow portion includes exactly twelve inflow crowns and exactly six outflow crowns.
